# EUROPEAN PATENT APPLICATION

(11) **EP 4 177 351 A1**
(43) Date of publication of application: **10.05.2023**
(21) Application number: 21206775.5
(22) Date of filing: 05.11.2021
(51) Int. Cl.: C12Q 1/04, G01N 1/22, C12Q 1/24

(54) **DEVICE AND METHOD FOR DETECTING THE PRESENCE OF MICROORGANISMS**

(71) Applicant: Airbus Operations GmbH, 21129 Hamburg (DE); Airbus SAS, 31700 Blagnac Cedex (FR)
(72) Inventor: SCHLIWA, Ralf, 21129 Hamburg (DE); THIBAUD, Catherine, 21129 Hamburg (DE); BEZOLD, Andreas, 21129 Hamburg (DE); ALONSO TABARES, Diego, 31700 Blagnac Cedex (FR); REIDT, Ulrich, 21129 Hamburg (DE); LOBENTANZER, Hans, 21129 Hamburg (DE); HELLER, Christoph, 21129 Hamburg (DE)
(74) Representative: Isarpatent

(57) **Abstract**

The present invention provides a device for detecting the presence of microorganisms (100) in a volume of air in an enclosed space, comprising a sampling device (110) configured to collect and store a sample from the volume of air; and an analyzing device (120) comprising a detection unit (121) configured to generate data concerning the presence of microorganisms in the sample stored in the sampling device (110). The present invention further provides a method for detecting the presence of microorganisms in a volume of air in an enclosed space.

## Description

The present invention is directed to a device for detecting the presence of microorganisms and a method for detecting the presence of microorganisms

The human body constantly sheds biological material, e.g. via the respiratory system. In the case that harmful microorganisms like bacteria or viruses are present in a person's body, said biological material might very well contain said harmful microorganisms. If another person is exposed to these shed microorganisms there is a distinct possibility that these microorganisms will settle in the other person's body leading to the spread of infectious diseases and the like.

This can prove especially troublesome in vehicles intended for mass transport, e.g. in commercial aircraft, as in these vehicles a large number of people spend relatively long periods of time within an enclosed space, increasing the risk of coming into contact with microorganisms shed by other people. Furthermore, some harmful microorganisms are not only transmitted via aerosols but can also settle on surfaces, posing a risk of transmission through contact with said surfaces even if the person shedding the microorganisms has already left the vehicle.

In order to minimize the risk of transmission of harmful microorganisms from one person to another, among other measures, the surfaces within a vehicle can be disinfected. Strictly speaking, such disinfection measurements are only necessary if harmful microorganisms are actually present on the surfaces to be disinfected. Performing disinfection measurements in the absence of harmful microorganisms leads to an increase in costs while operating a vehicle like a commercial aircraft.

Document DE 10 2008 035 771 B4 shows device for the automatic detection of microorganisms. However, the need to transport samples potentially containing microorganisms into separate vessels for analysis inhibits the potential of said device in the day-to-day usage of for example commercial aircraft travel, as this leads to an increase of required space and expertise of users of the device.

The present invention therefore seeks to provide a way of quickly and reliably detecting the presence of microorganisms in order to save money and material needed for disinfection measurements without unduly hazarding the transmission of harmful microorganisms.

According to the present invention, this problem is solved by a device for detecting the presence of microorganisms with the features of patent claim 1 and a method for detecting the presence of microorganisms with the features of patent claim 11.

For that purpose, a device for detecting the presence of microorganisms in a volume of air in an enclosed space is presented. The device comprises a sampling device and an analyzing device. The sampling device is configured to collect and store a sample from the volume of air. The analyzing device comprises a detection unit configured to generate data concerning the presence of microorganisms in the sample stored in the sampling device.

Furthermore, a method for detecting the presence of microorganisms in a volume of air in an enclosed space is presented. A sample from the volume of air is collected and stored with a sampling device. Data concerning the presence of microorganisms in the sample stored in the sampling device is generated with a detection unit of an analyzing device.

It is an idea of the present invention to collect and analyze samples of the air in an enclosed space in order to continuously determine whether microorganisms are present within the air of the enclosed space and by extension on surfaces of the enclosed space. This provides up-do-date information on whether further measurements, like disinfecting or even evacuating the enclosed space are necessary or not. Having information that is more accurate allows for more efficient application of measurements to prevent transmission of harmful microorganisms and therefore can increase the safety of people in the enclosed space and simultaneously lower efforts and costs expended on unnecessary measurements.

Further embodiments and developments are defined in the dependent claims.

According to a further development, the sampling device comprises an inlet configured to allow air to enter the sampling device, a separating unit configured to separate microorganisms from the air entering the sampling device, an outlet configured to allow air to exit the sampling device, and a collection vessel configured to store the microorganisms separated from the air. This concept for sampling devices, often referred to as "impingers", is well known and tested, advantageously providing a sure method of collecting and storing samples to be analyzed.

According to a further development, the detection unit is configured to generate data without contacting the sample. In this way, there is no need for disinfecting, cleaning, or resetting the detection unit, advantageously allowing for almost continuous updates on the presence of microorganisms.

According to a further development, the detection unit comprises at least one of a multi-dimensional non-linear spectrographic system or a multicapillary column coupled ion mobility spectrometry system. These are particularly advantageous examples of contactless techniques used to reliably detect microorganisms.

According to a further development, the detection unit is configured to analyze the sample while directly contacting the sample. Depending on the microorganisms to be detected, a contacting method of detecting can be more reliable than contactless methods. In this case, the reliable detection may advantageously outweigh the need to regularly clean or disinfect the detecting unit.

According to a further development, the sampling device comprises a disinfection unit configured to remove the sample from the sampling device and prepare the sampling device for collecting a new sample. In order to monitor the presence of microorganisms, it can be advantageous to reset the sampling device at certain intervals and this is advantageously done via an integrated automatic mechanism in order to lower the workload of handlers for the device.

According to a further development, the analyzing device comprises a computation unit configured to evaluate the data generated by the detection unit. Advantageously, the analyzing device not only generates data concerning the presence of microorganisms but also contains the means to independently and automatically analyze said data in order to facilitate decisions on what measurements should be taken according to the results of the analysis.

According to a further development, an output device is provided which is configured to initiate further steps based on the evaluation by the computation unit, in particular configured to display the results of the evaluation and/or to trigger a disinfection procedure for the enclosed space. Further automation of the disinfection process and other measures can advantageously reduce the workload of the operators, depending on the specific nature of the enclosed space and the relevant microorganisms.

The device according to the invention can be either integrated into a portable device for detecting the presence of microorganisms in a volume of air or fixedly installed in a vehicle. Portable devices can advantageously be used to collect samples from different positions in an enclosed space, while fixedly installed devices can be, depending on circumstance, easier calibrated and therefore provide results that are more reliable.

The above embodiments and further developments can be combined as desired or useful. Further possible embodiments, further developments and implementations of the invention also comprise combinations of features of the invention described above or below which are not explicitly mentioned. In particular, the person skilled in the art will also add individual aspects as improvements or additions to the respective basic form of the present invention.

The present invention is explained in more detail below with reference to the embodiments shown in the schematic Figures. The following are shown:
- Fig. 1: a device for detecting the presence of microorganisms according to an embodiment of the present invention;
- Fig. 2: a device for detecting the presence of microorganisms according to another embodiment of the present invention;
- Fig. 3: a sampling device that is part of an embodiment of a device for detecting the presence of microorganisms;
- Fig. 4: a vehicle incorporating embodiments of devices for detecting the presence of microorganisms;
- Fig. 5: a method for detecting the presence of microorganisms according to an embodiment of the present invention; and
- Fig. 6: a method for detecting the presence of microorganisms according to an embodiment of the present invention.

The accompanying figures are intended to provide a further understanding of embodiments of the invention. They illustrate embodiments and, in connection with the description, serve to explain principles and concepts of the invention. Other embodiments and many of the advantages mentioned will be apparent with reference to the drawings. The elements of the drawings are not necessarily shown to scale with respect to each other.

In the figures of the drawings, identical elements, features and components with the same function and the same effect are marked with the same reference signs, unless otherwise specified.

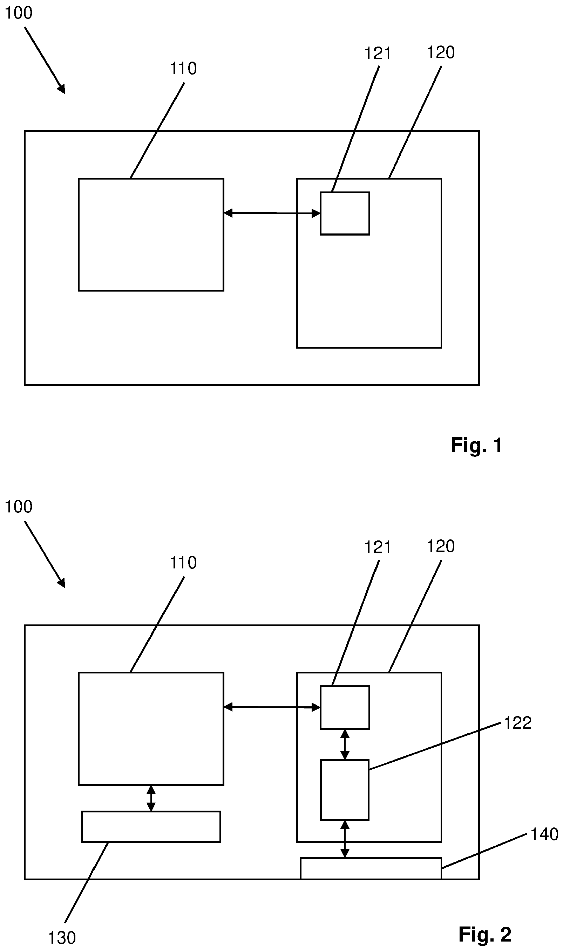

Figure 1 shows a device for detecting the presence of microorganisms 100 in a volume of air in an enclosed space. The device for detecting the presence of microorganisms 100 comprises a sampling device 110 and an analyzing device 120. The analyzing device 120 further comprises a detection unit 121.

A sample of the air in the enclosed space to be analyzed is collected and stored in the sampling device 110. The detection unit 121 then generates data concerning the presence of microorganisms in the sample stored in the sampling device 110. The presence of microorganisms in the sample is representative of the presence of microorganisms in the volume of air from which the sample has been drawn. In this way, the device for detecting the presence of microorganisms 100 can be used to determine whether and to what extent microorganisms are present in the enclosed space and whether and which measures need to be taken accordingly.

In the device shown in Figure 1, the detection unit generates the data without contacting the sample stored in the sampling device. This can be accomplished with a variety of technical implementations, depending on which microorganisms are relevant for detection.

Multi-dimensional non-linear spectrographic systems (MNLS) use cell communication physics to identify a resonance in a body stimulated by specific frequency spectra. As an example, the specific frequency signature of a microorganism can be digitized and stored in a database. Subsequently, transmitting this frequency to the sample allows identification and location of the specific microorganism load. Such a test can for example be done in about 10 seconds.

Along with microorganisms, volatile organic compounds can be released by the metabolism of the microorganism or its host. Such volatile organic compounds will be collected along with the microorganisms themselves by the sampling device, and can then be detected for example vial ion mobility spectrometry, providing another contactless avenue for determining whether microorganisms are presented in the sample and by extension in the enclosed space.

In general, the method of generating the data concerning the presence of microorganisms in the sample will strongly depend on the type and species of microorganism to be detected. A multitude of contacting and contactless techniques exists for this purpose and the skilled person will be able to choose the technique best suited for the specific application of the device shown.

Figure 2 shows a device for detecting the presence of microorganisms 100 with the features shown in the embodiment of Figure 2. The embodiment of Figure 2 further comprises a disinfecting device 130 and an output device 140. The analyzing device shown in Figure 2 further comprises a computation unit 122.

Providing the device with these additional features allows for further automation of the detection process as supplementary measures such as disinfecting the sampling device and deciding on further measures like evacuating and/or disinfecting the enclosed space can be performed by the device itself instead of requiring action by auxiliary personnel.

Of course, said supplementary measures can be performed manually or at the least be supervised by human personnel, trading an increase in workload for additional security against technical complications occurring in the device.

In this embodiment, the device for detecting the presence of microorganism 100 comprises a computation unit 122 for analyzing the data generated by the detection unit 121. In another embodiment, the detection unit 121 may be configured to transmit the generated data to an external computation unit, in particular via a wireless connection. This can be preferable, as this way the device for detecting the presence of microorganisms 100 can be optimized for restrictions in available space. Such an external computation unit may be located on a vehicle with the device for detecting the presence of microorganisms 100 or may be located outside such a vehicle, for example in an operations center.

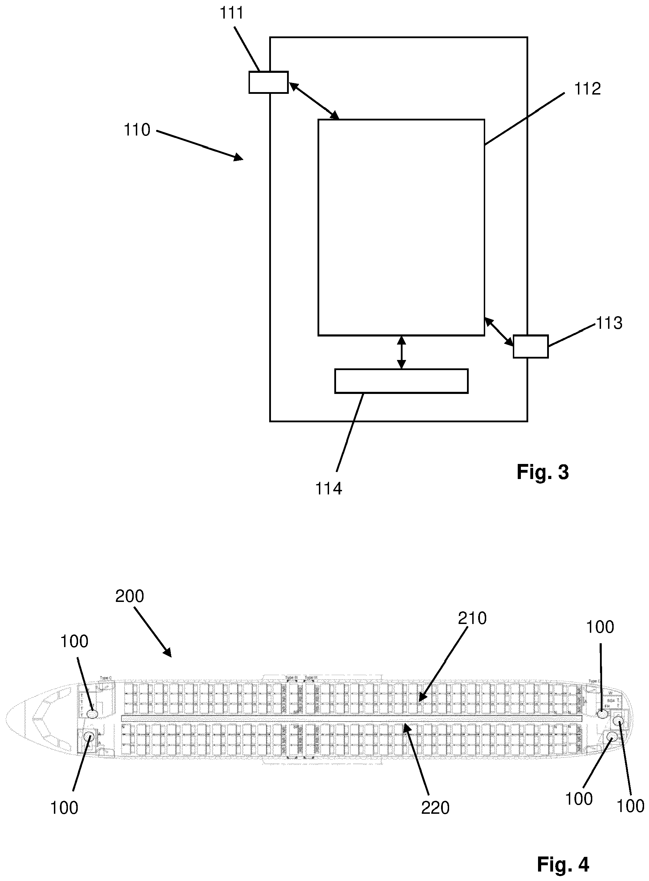

Figure 3 shows a sampling device 110 of an embodiment of a device for detecting the presence of microorganisms. The sampling device comprises an inlet 111, a separating unit 112, an outlet 113, and a collection vessel 114.

In this embodiment, air from the enclosed space continuously passes through the sampling device 110, in particular through the separating unit 111, where microorganisms are separated from the airflow and transported into the collection vessel 114. This process increases the concentration of microorganisms in the collection vessel 114 over a period of time, allowing the application of detection methods which require a certain minimum concentration of microorganisms to provide reliable results.

Figure 4 shows a vehicle 200, a commercial aircraft. The vehicle 200 comprises an enclosed space 210, a cabin. An area 220 is shown where a portable device for detecting the presence of microorganisms can be moved through. Several devices for detecting the presence of microorganisms 100 are arranged within the enclosed space 210.

In this embodiment, two embodiments of devices for detecting the presence of microorganisms can work in tandem. A portable device can be moved through the area 220, which is here for example the aisle of a cabin in a passenger aircraft. This allows for collecting a sample representative of the enclosed space as a whole even in the absence of extensive air circulation through the enclosed space, which is generally not present in the cabin of an aircraft. For this purpose, the portable device is advantageously stored in a housing with the dimensions of a standard unit fitting into for example a trolley of the kind flight crew personnel is moving through the aisle in the process of serving passengers.

In addition, further devices for detecting the presence of microorganisms can be fixedly installed on crucial points, e.g. the entrances and at the lavatories, where multiple persons will pass through or linger during travel. The detection of a presence of microorganisms in these places, where several people spend time, is especially beneficial as the risk of transmission is higher at those places than elsewhere in the cabin.

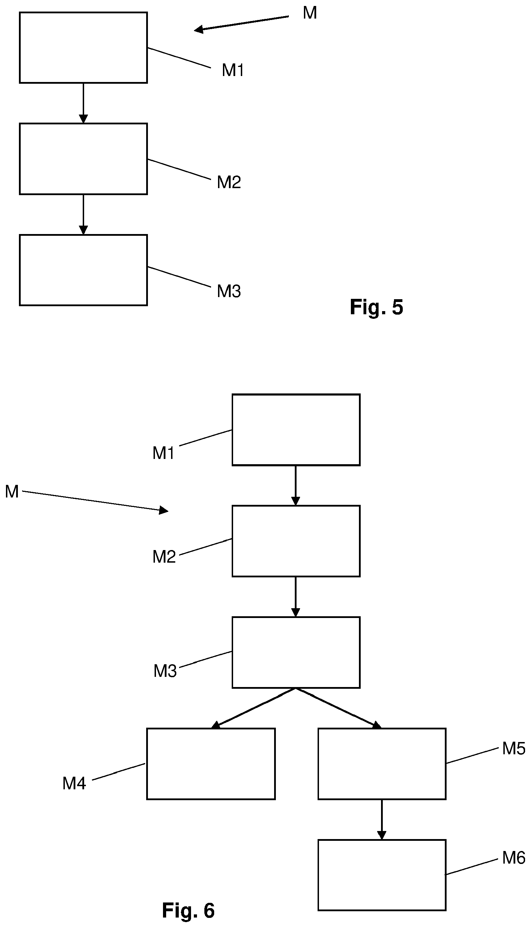

Figure 5 shows a flow diagram for a method for detecting the presence of microorganisms M. In a first step M1, a sample of air is collected by a sampling device. In a second step M2, the sample is stored in the sampling device. In a third step M3, data concerning the presence of microorganisms in the sample is generated by a detection unit of an analyzing device.

Figure 6 shows a flow diagram for a method for detecting the presence of microorganisms M. In addition to the steps shown in Figure 5, after the step of generating data M3, the sample is removed from the sampling device and the sampling device is prepared for collecting a new sample in a fourth step M4. In addition or alternatively to step M4, the data generated in step M3 is analyzed by a computation unit of the analyzing device in a fifth step M5. In a sixth step M6, further measures are taken depending on the evaluation performed in step M5.

Above, the present invention has been described with a focus on applications within a vehicle, in particular in an aircraft. The present invention can, however be applied in various locations, in particular wherever multiple people spend an extended amount of time in an essentially enclosed space. In particular the present invention can be utilized in various other vehicles, in particular buses and trains, as well as buildings, in particular airports. The skilled person will understand that depending on the enclosed space, in which the present invention is used, further measures in reaction to the presence of microorganisms, e.g. disinfection and/or evacuation, will differ accordingly.

### List of reference signs

- 100: device for detecting the presence of microorganisms
- 110: sampling device
- 111: inlet
- 112: separating unit
- 113: outlet
- 114: collection vessel
- 120: analyzing device
- 121: detection unit
- 122: computation unit
- 130: disinfection device
- 140: output device
- 200: vehicle
- 210: enclosed space
- 220: area
- M: method for detecting the presence of microorganisms
- M1: step of collecting sample
- M2: step of storing sample
- M3: step of generating data
- M4: step of disinfecting
- M5: step of analyzing step
- M6: step of performing further measures

## Claims

1. Device for detecting the presence of microorganisms (100) in a volume of air in an enclosed space (210), comprising:
a sampling device (110) configured to collect and store a sample from the volume of air; and
an analyzing device (120) comprising a detection unit (121) configured to generate data concerning the presence of microorganisms in the sample stored in the sampling device (110).

2. Device for detecting the presence of microorganisms (100) according to claim 1, wherein the sampling device (110) comprises
an inlet (111) configured to allow air to enter the sampling device;
a separating unit (112) configured to separate microorganisms from the air entering the sampling device;
an outlet (113) configured to allow air to exit the sampling device; and
a collection vessel (114) configured to store the microorganisms separated from the air.

3. Device for detecting the presence of microorganisms (100) according to claim 1 or claim 2, wherein the detection unit (121) is configured to generate data without contacting the sample.

4. Device for detecting the presence of microorganisms (100) according to claim 3, wherein the detection unit (121) comprises at least one of a multi-dimensional non-linear spectrographic system or a multicapillary column coupled ion mobility spectrometry system.

5. Device for detecting the presence of microorganisms (100) according to claim 1 or claim 2, wherein the detection unit (121) is configured to analyze the sample while directly contacting the sample.

6. Device for detecting the presence of microorganisms (100) according to any one of the preceding claims, further comprising a disinfection unit (130) configured to remove the sample from the sampling device (110) and prepare the sampling device (110) for collecting a new sample.

7. Device for detecting the presence of microorganisms (100) according to any one of the preceding claims, wherein the analyzing device (120) comprises a computation unit (122) configured to evaluate the data generated by the detection unit (121).

8. Device for detecting the presence of microorganisms (100) according to claim 7, further comprising an output device (140) configured to initiate further steps based on the evaluation by the computation unit (122), in particular configured to display the results of the evaluation and/or to trigger a disinfection procedure for the enclosed space.

9. Portable device for detecting the presence of comprising a housing and a device for detecting the presence of microorganisms (100) according to any of the preceding claims arranged in the housing.

10. Vehicle (200) comprising an enclosed space (210) and a device for detecting the presence of microorganisms (100) according to any one of claims 1 to 8 arranged in the enclosed space (210).

11. Method for detecting the presence of microorganisms (M) in a volume of air in an enclosed space, the method comprising the steps of:
collecting (M1) and storing (M2) a sample from the volume of air with a sampling device; and
generating data (M3) concerning the presence of microorganisms in the sample stored in the sampling device with a detection unit of an analyzing device.

12. Method for detecting the presence of microorganisms (M) according to claim 11, wherein the step of generating data (M3) is performed without the detection unit being in contact with the sample.

13. Method for detecting the presence of microorganisms (M) according to claim 11 or claim 12, further comprising the step of
removing (M4) the sample from the sampling device and preparing the sampling device for collecting a new sample.

14. Method for detecting the presence of microorganisms (M) according to any one of claims 11 to 13, further comprising the step of
evaluating (M5) the data generated by the detection unit with a computation unit of the analyzing device.

15. Method for detecting the presence of microorganisms (M) according to claim 14, further comprising the step of
taking further measures (M6), in particular disinfecting the enclosed space, based on the evaluation (M5) by the computation unit.
